# EUROPÄISCHE PATENTSCHRIFT

(11) **EP 3 668 590 B1**
(45) Veröffentlichungstag und Bekanntmachung des Hinweises auf die Patenterteilung: **27.09.2023**
(21) Anmeldenummer: 18755436.5
(22) Anmeldetag: 10.08.2018
(51) Int. Cl.: A61N 1/375, A61N 1/05, A61N 1/36, A61B 5/00, H01L 23/31, H01L 23/498, H01L 25/00

(54) **ISOLATION EINER PASSIVIERUNG**
ISOLATION OF A PASSIVATION
ISOLATION D'UNE PASSIVATION

(30) Priorität: 16.08.2017 DE 102017118687
(43) Veröffentlichungstag der Anmeldung: 24.06.2020
(73) Patentinhaber: Aesculap AG, 78532 Tuttlingen (DE); Eberhard Karls Universität Tübingen, 72074 Tübingen (DE)
(72) Erfinder: HOFMANN, Boris, 78582 Balgheim (DE); WESTERHAUSEN, Markus, 72762 Reutlingen (DE)
(74) Vertreter: Winter, Brandl - Partnerschaft mbB
(86) Internationale Anmeldenummer: PCT/EP2018/071772
(87) Internationale Veröffentlichungsnummer: WO 2019/034558

(56) Entgegenhaltungen:
- WO-A1-2011/155946
- WO-A1-2016/074974
- US-A1- 2012 112 347

## Beschreibung

Die Erfindung betrifft eine Isolation einer künstlich geschaffenen Öffnung in einer Passivierung, und bezieht sich insbesondere auf ein elektrisch und/oder fluiddicht und/oder gasdicht isolierendes Verschließen einer in vorbestimmter Weise in einer Passivierung erzeugten Öffnung, ohne diese Öffnung vollständig beispielsweise galvanisch aufzufüllen und/oder vollständig zu verschließen.

Für medizinische Therapie- und Diagnoseverfahren sind beispielsweise elektrisch aktive und/oder flexible Implantate bekannt. Solche Implantate sind in der Regel biostabil, biokompatibel sowie elektrisch isolierend verkapselt und passiviert, beispielsweise mittels einer Passivierungsschicht oder eines unter anderem mehrere Passivierungsschichten und zumindest eine zwischen diesen angeordnete, elektrisch ankontaktierte Zwischenlage aufweisenden Mehrschicht-Passivierungssystems.

In verkapselten, flexiblen Implantaten können Öffnungen in einer Passivierung einen elektrischen Zugang zu der zumindest einen elektrisch aktiven Zwischenlage in dieser Passivierung bzw. zwischen Passivierungsschichten oder Passivierungslagen ermöglichen, und ist häufig auch die Passivierung über einer Elektrode bzw. einer Implantatelektrode mittels einer künstlich, d.h. in einer vorbestimmten Weise, geschaffenen oder erzeugten Öffnung geöffnet, um einen elektrischen Kontakt zwischen der Elektrode und dem Gewebe herzustellen.

Die künstlich geschaffene Öffnung lässt jedoch die Seitenwand der Öffnung vulnerabel gegenüber elektrischen wie auch negativ degradierenden Einflüssen. Der künstlich in Form der Öffnung geschaffene Defekt in der Passivierung ist zwar durch beispielsweise galvanisches Auffüllen mit elektrisch leitfähigem Material wieder verschließbar, wonach die Elektrodenseitenwand wieder gegenüber Degradation bzw. Beeinträchtigung (Nässe, Delamination usw.) geschützt ist und auch die Elektrode weiterhin mit dem Gewebe kontaktiert ist.

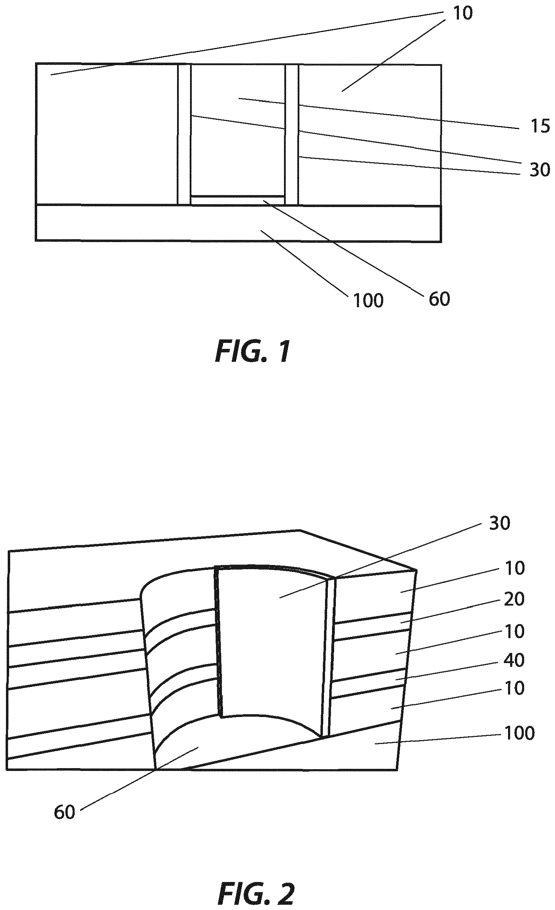

Allerdings ist nach dem galvanischen Auffüllen mit elektrisch leitfähigem Material auch eine Leitfähigkeit gegenüber der Seitenwand in der Passivierung gegeben, sodass weitere elektrische aktive Lagen unweigerlich ebenfalls elektrisch mit der Elektrode verbunden sind (vgl. Fig. 2, linke Bildhälfte mit elektrisch aktiven Flächen leitfähigen Materials, beispielsweise Metallflächen, in einer Passivierung, die bei galvanischer Auffüllung gegenüber der Elektrode über beispielsweise ein Fluid elektrisch kontaktiert wären). Derartige (notwendigerweise) geschaffene Öffnungen in der Passivierung ermöglichen somit nachteilig auch einen unerwünschten elektrischen Zugang zu der zumindest einen elektrisch aktiven Zwischenlage in der Passivierung.

US 2012/112347 A1 und WO 2011/155946 A1 offenbaren eine auf einen elektrischen Leiter aufgebrachte, eine Öffnung aufweisende Kunststoffschicht, wobei auf die Kunststoffschicht - auch im Bereich der Öffnung - eine DLC-Beschichtung aufgebracht ist. Dabei wird die beispielsweise mittels plasmaunterstützter chemischer Gasphasenabscheidung aufgebrachte DLC-Beschichtung als Passivierungsschicht bezeichnet.

Die Erfindung liegt als eine Aufgabe zugrunde, ein Verfahren und ein Passivierungssystem bereitzustellen, mittels welchen eine Öffnung in einer Passivierung gegenüber der Passivierung zumindest elektrisch isolierbar ist, und welche ermöglichen, dass elektrisch aktive Bauteile innerhalb der Passivierung weiter genutzt werden können.

Diese Aufgabe wird erfindungsgemäß durch ein Verfahren zur Isolation einer Passivierung mit den Merkmalen des Anspruchs 1 und ein Passivierungssystem mit den Merkmalen des Anspruchs 5 gelöst. Vorteilhafte Weiterbildungen der Erfindung sind Gegenstand der beigefügten Unteransprüche.

Der Erfindung liegt die allgemeine Idee zugrunde, eine künstliche geschaffene Öffnung in einer passivierenden Schicht nicht (nur) mittels beispielsweise galvanischer Auffüllung zu schließen, sondern diese Öffnung mit einer wenigstens elektrisch isolierenden Seitenwand wieder zu verschließen, ohne sie dabei komplett verschließen oder wieder auffüllen zu müssen. In anderen Worten wird erfindungsgemäß anstelle des bekannten galvanischen Auffüllens und/oder Verschlie-ßens der Öffnung die Umfangswandung der Öffnung mit einer elektrisch und/oder fluiddicht und/oder gasdicht isolierenden Seitenwandung oder Beschichtung versehen, die verhindert, dass etwaige elektrisch aktive Komponenten in der Passivierung gegenüber einem über die im Übrigen frei bleibende Öffnung die Elektrode des Implantats beaufschlagenden Elektrolyten (Fluid) ankontaktiert werden. Die erfindungsgemäß vorgeschlagene Seitenwandpassivierung stellt somit nicht nur eine (elektrische und/oder fluiddichte und/oder gasdichte) Isolierung gegenüber der Passivierung dar, sondern schützt diese zusätzlich gegen weitere degradierende Effekte wie einem direkten Aussetzen und/oder Freiliegen von Passivierungslagen und Zwischenlagen bzw. entsprechenden Teilen des Mehrschichtsystems gegenüber Flüssigkeit. Zugleich wird die Passivierung gegen seitliches Eindringen von Fluiden wie beispielsweise Wasser geschützt. Die Öffnung selbst in der Passivierung bleibt bestehen und kann für weitere Anwendungen, beispielsweise für eine Durchführung, eine Steckverbindung oder einen Header, genutzt werden, oder bedarfsweise galvanisch aufgefüllt werden. Durch das Beschichten der Seitenwandung(en) von (Elektroden-) Öffnungen mit einem elektrisch isolierenden Layer kann die eigentliche Passivierungsschicht ebenfalls elektrisch genutzt werden, da diese isoliert von einer künstlich geschaffenen (Elektroden-) Öffnung arbeitet, gleichzeitig aber auch gegenüber äußeren Einflüssen geschützt ist, die aufgrund dieser Öffnungen einwirken können.

Im Einzelnen beinhaltet ein Verfahren zur Isolation einer Passivierung einen Schritt, bei dem entlang des Umfangs zumindest einer in vorbestimmter Weise geschaffenen Öffnung in der Passivierung eine isolierende Wandung angeordnet wird.

Dabei wird die isolierende Wandung elektrisch isolierend und/oder fluiddicht und/oder gasdicht isolierend erzeugt.

Bevorzugt erstreckt sich die isolierende Wandung von einem unteren Ende der Passivierung durch die Passivierung hindurch zu einem oberen Ende der Passivierung hin.

In dem vorstehenden Verfahren wird die isolierende Wandung in zumindest einer rundförmigen, rechteckförmigen, elliptischen oder konusförmigen Öffnung und vorzugsweise die gesamte Seitenwandungsfläche der Öffnung in der Passivierung bedeckend aufbeschichtet, wobei die Passivierung eine einschichtige Passivierung oder ein mehrschichtiges Passivierungssystem mit zumindest einer elektrisch aktiven Zwischenlage ist, und isoliert die isolierende Wandung die zumindest eine elektrisch aktive Zwischenlage und/oder zumindest eine Elektrode eines flexiblen oder starren biostabilen, biokompatiblen und elektrisch isolierend verkapselten medizinischen Implantats als eine Bodenfläche der Öffnung elektrisch und/oder fluiddicht und/oder gasdicht.

Bevorzugt wird in dem Verfahren die isolierende Wandung mit einer vorbestimmten Dicke erzeugt und dazu konfiguriert, in der Öffnung einen Öffnungskanal eines vorbestimmten Durchmessers in der Passivierung für eine Fluidzuleitung zu einer Elektrode und/oder für eine Aufnahme einer Durchführung, einer Steckverbindung und/oder einer Headervorrichtung zu erhalten und gleichzeitig elektrisch aktive Komponenten in der Passivierung gegenüber dem Öffnungskanal elektrisch isoliert zu halten und/oder gegen ein Eindringen des Fluids in die Passivierung zu schützen.

Gemäß einem weiteren Aspekt der Erfindung beinhaltet ein Passivierungssystem eine elektrisch und/oder fluiddicht und/oder gasdicht isolierend erzeugte isolierenden Wandung entlang des Umfangs zumindest einer in vorbestimmter Weise geschaffenen Öffnung in der Passivierung.

Dabei erstreckt sich die isolierende Wandung von einem unteren Ende der Passivierung durch die Passivierung hindurch zu einem oberen Ende der Passivierung.

Die isolierende Wandung ist in zumindest einer rundförmigen, rechteckförmigen, elliptischen oder konusförmigen Öffnung und die gesamte Wandungsfläche der Öffnung in der Passivierung bedeckend angeordnet, wobei die Passivierung eine einschichtige Passivierung oder eine mehrschichtiges Passivierungssystem mit zumindest einer elektrisch aktiven Zwischenlage ist und die isolierende Wandung dazu angeordnet ist, die zumindest eine elektrisch aktive Zwischenlage und/oder zumindest eine Elektrode eines flexiblen oder starren biostabilen, biokompatiblen und elektrisch isolierend verkapselten medizinischen Implantats als eine Bodenfläche der Öffnung elektrisch und/oder fluiddicht und/oder gasdicht zu isolieren.

Bevorzugt ist die isolierende Wandung mit einer vorbestimmten Dicke erzeugt und dazu konfiguriert, in der Öffnung einen Öffnungskanal eines vorbestimmten Durchmessers in der Passivierung für eine Fluidzuleitung zu der Elektrode und/oder eine Aufnahme einer Durchführung, einer Steckverbindung und/oder einer Headervorrichtung zu erhalten und gleichzeitig elektrisch aktive Komponenten in der Passivierung gegenüber dem Öffnungskanal elektrisch isoliert zu halten und/oder gegen ein Eindringen des Fluids in die Passivierung zu schützen.

Die Erfindung wird nachstehend mit weiteren Vorteilen und Wirkungen anhand bevorzugter Ausführungsbeispiele unter Bezugnahme auf die Zeichnung näher beschrieben. Es zeigen:
Fig. 1 auszugsweise und schematisch eine Passivierung für beispielsweise ein medizinisches Implantat mit einer künstlich geschaffenen Öffnung und einer in der Öffnung angeordneten elektrisch isolierenden Seitenwand gemäß einem Ausführungsbeispiel;
Fig. 2 auszugsweise und schematisch ein dreidimensionales Schema der Seitenwandpassivierung gemäß dem Ausführungsbeispiel mit elektrisch aktiven Flächen in der Passivierung in einem Mehrschichtsystem;
Fig. 3 ein Diagramm zur Darstellung eines Nachweises einer isolierenden Seitenwand gemäß dem Ausführungsbeispiel
Fig. 4 auszugsweise und schematisch ein Beispiel einer Steckverbindung (Header) mit einer Seitenwandpassivierung; und
Fig. 5 ein Beispiel eines Substrats mit geöffneten Elektroden und Lagen zumindest elektrisch leitfähigen Materials in der Passivierung gemäß dem Ausführungsbeispiel.

Gleiche oder funktionell äquivalente Merkmale sind in den einzelnen Figuren mit denselben Bezugszeichen versehen und werden zweckmäßig nicht redundant beschrieben. Figuren sind nicht notwendigerweise als maßstäblich zu betrachten. Eine Beschränkung auf in den Figuren angegebene Maßeinheiten besteht nicht.

Fig. 1 zeigt auszugsweise und schematisch vereinfacht eine Passivierung für beispielsweise ein medizinisches Implantat mit einer künstlich geschaffenen Öffnung und einer in der Öffnung angeordneten elektrisch isolierenden Seitenwand gemäß einem Ausführungsbeispiel.

Wie in Fig. 1 gezeigt, ist auf einem Substrat 100, das Teil des nicht weiter dargestellten Implantats bilden kann, eine Elektrode bzw. Implantatelektrode 60 und diese umgebend eine Passivierung 10 angeordnet. Die Elektrode 60 ist darüber hinaus in der künstlich, d.h. in einer vorbestimmten Weise, in der Passivierung 10 geschaffenen (Elektroden-) Öffnung 15 angeordnet und bildet in dieser eine Bodenfläche aus. An dem Umfang bzw. der (Innen-) Wandung der Öffnung 15 ist, innerhalb der Öffnung 15 und ausgehend von der Elektrode 60 bzw. einer unteren Seite bzw. einem unteren Ende der Passivierung 10, eine sich zu einer oberen Seite bzw. einem oberen Ende der Passivierung 10 hin erstreckende isolierende Schicht 30 mit einer vorbestimmten Dicke erzeugt.

Form und die Größe der Öffnung 15 sind in diesem Ausführungsbeispiel nicht auf eine bestimmte Anzahl, Form und Größe beschränkt, sondern diese Parameter können anwendungsspezifisch festgelegt sein. Beispielsweise kann eine Öffnung 15 angeordnet sein, oder können mehrere Öffnungen 15 gleicher oder unterschiedlicher Größe in gleichen oder unterschiedlichen Abständen zueinander angeordnet sein. Die Öffnung selbst kann beispielsweise rundförmig bzw. zylindrisch, rechteckförmig einschließlich quadratisch, elliptisch oder nach oben oder unten konisch zulaufend ausgebildet sein. Es versteht sich, dass anwendungsspezifisch darüber hinaus noch weitere geeignete Öffnungsformen denkbar sein können.

Auch bezüglich des Verfahrens der Anbringung der isolierenden Schicht 30 bestehen gemäß diesem Ausführungsbeispiel keine besonderen Beschränkungen, solange es sich um ein für eine beabsichtigte Verwendung oder Anwendung, beispielsweise solche aus dem Bereich medizinischer Implantate, zugelassenes und/oder kompatibles und insgesamt geeignetes Auftrags- oder Beschichtungsverfahren handelt.

Die isolierende Schicht 30 selbst ist vorteilhaft und bevorzugt zumindest elektrisch isolierend, und/oder fluiddicht isolierend und/oder gasdicht isolierend. In einer Modifikation können Eigenschaften dieser Art ortspezifisch erzeugt oder variiert (verstärkt oder verringert) sein, d.h. beispielsweise über einen Mehrfachauftrag entsprechender Schichten und/oder eine Dickenvariation der isolierenden Schicht 30 entlang ihrer Erstreckungslänge. Insgesamt bildet die isolierende Schicht 30 eine zumindest elektrisch isolierende Seitenwand bzw. elektrische Seitenwandisolierung in der künstlich geschaffenen Öffnung 15 in der Passivierung 10 aus und resultiert in einer Passivierung mit elektrisch passivierter Seitenwand.

Fig. 2 zeigt auszugsweise und schematisch ein dreidimensionales Schema der Seitenwandpassivierung gemäß dem Ausführungsbeispiel mit einer isolierenden Barriere gegenüber elektrisch aktiven Flächen innerhalb der passivierenden Schicht bzw. in der Passivierung in einem Mehrschichtsystem.

Im Einzelnen zeigt Fig. 2 einen Ausschnitt eines mehrlagigen Passivierungssystems auf dem Substrat 100 mit der durch das Passivierungssystem vor vielfältigen negativen Degradationseffekten geschützten (Implantat-) Elektrode 60. Das mehrlagige Passivierungssystem bildet mit seinen Passivierungsschichten bzw. Passivierungslagen 10, hier einer oberen bzw. ersten, einer mittleren bzw. zweiten und einer unteren bzw. dritten Passivierungsschicht, und dazwischen liegenden, elektrisch aktiven Zwischenlagen 20, 40, hier einer oberen bzw. ersten Zwischenlage 20 (Autop) und einer unteren bzw. zweiten Zwischenlage 40 (Aubottom) aus einem elektrisch leitfähigem Material, beispielsweise einem Metall oder Halbleiter, ein passivierendes Mehrschichtsystem aus. Fig. 2 zeigt ebenfalls die Öffnung 15 (das Bezugszeichen ist in der Figur aus Gründen besserer Übersicht weggelassen) und einen Teilabschnitt der isolierenden Wandung 30 am Umfang der Öffnung 15.

Die in Fig. 2 dargestellte Seitenwandpassivierung stellt nicht nur eine (elektrische) Isolierung gegenüber der Passivierung dar, sondern schützt diese zusätzlich gegen weitere degradierende Effekte wie beispielsweise einer direkten Beaufschlagung durch sich in der Öffnung 15 befindende Flüssigkeit. Wie aus Fig. 2, linker Teil, ersichtlich wäre eine ungeschützte Seitenwand in der Passivierung bei konventioneller (galvanischer) Auffüllung des in Form der Öffnung 15 künstlich geschaffenen Defekts in der Passivierung unweigerlich ebenfalls elektrisch mit der Elektrode 60 verbunden.

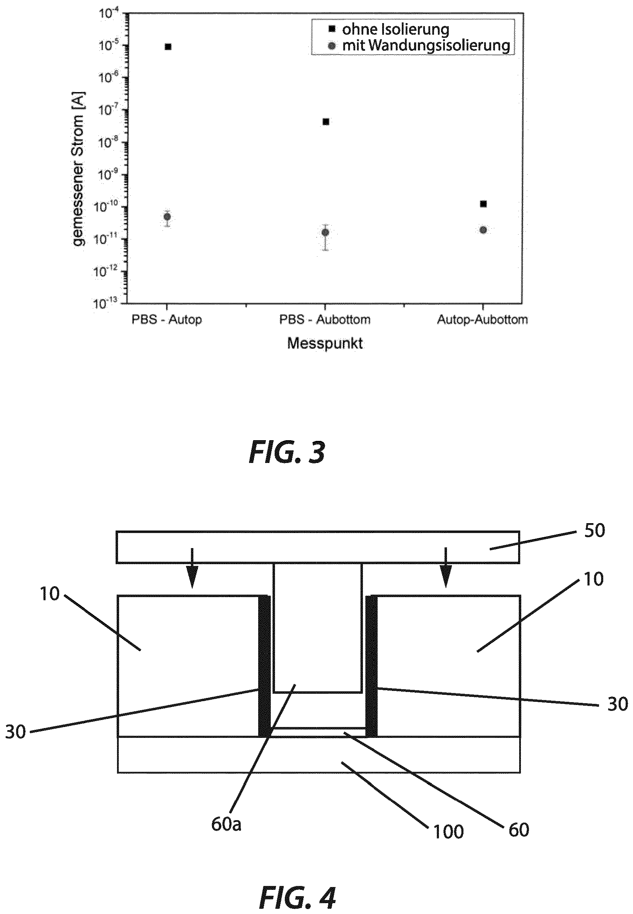

Fig. 3 zeigt ein Diagramm zur Darstellung eines Nachweises einer Funktion der isolierenden Seitenwand gemäß dem Ausführungsbeispiel. Für einen Funktionsnachweis wurde in diesem Ausführungsbeispiel ein Strom (Gleichstrom, 1 V angelegt; damit direkt reziprok zum Widerstand) von einer (nicht gezeigten) Referenzelektrode, die in einem (nicht gezeigten) PBS-Bad über der ungeschützten, d.h. keine isolierende Wandung aufweisenden Elektrodenöffnung 15 gelagert war, gegen die beiden Zwischenlagen 20, 40 (Autop, Aubottom; vgl. Fig. 2) aus leitfähigem Material gemessen. Sodann wurde die Seitenwand der Elektrodenöffnung 15 mit Hilfe einer physikalischen Atomlagenabscheidung mit einer 100 nm dicken Ti02-Schicht elektrisch isoliert und die vorstehend beschriebene Messung erneut durchgeführt. PBS steht für beispielsweise Phosphate Buffer Solution bzw. Phosphate Buffered Saline, d.h. eine phosphatgepufferte Salzlösung. PBS und deren Anwendbarkeit sind insoweit bekannt und wird daher hierin nicht redundant beschrieben.

Im Ergebnis ist aus Fig. 3 zu ersehen, dass der elektrische Widerstand nach Aufbringen bzw. Aufbeschichten der Isolierung auf die Wandung der Elektrodenöffnung 15 um Größenordnungen zunimmt und entsprechend der bei Vorhandensein der isolierenden Wandung gemessene Strom (untere Punktreihe in Fig. 3) gegenüber der nicht isolierten Wandung auf Werte von ca. 10⁻¹⁰ bis 10⁻¹¹ A abnimmt. Bei solchen Werten ist die Seitenwand als elektrisch isoliert anzusehen.

Fig. 4 zeigt auszugsweise und schematisch ein Beispiel einer Steckverbindung (Header) mit einer Seitenwandpassivierung. Da gemäß dem vorliegenden Ausführungsbeispiel in der Passivierung 10 eine lediglich um die Dicke der isolierenden Wandung bzw. Beschichtung reduzierte Öffnung 15 erhalten bleibt, kann diese Öffnung 15 für weitere Anwendungen genutzt werden, beispielsweise für eine Durchführung, eine Steckverbindung oder einen Header 50 mit einem, angedeutet durch Pfeile in Fig. 4, in die Öffnung 15 einführbaren und die Elektrode 60 kontaktierenden Abschnitt 60a.

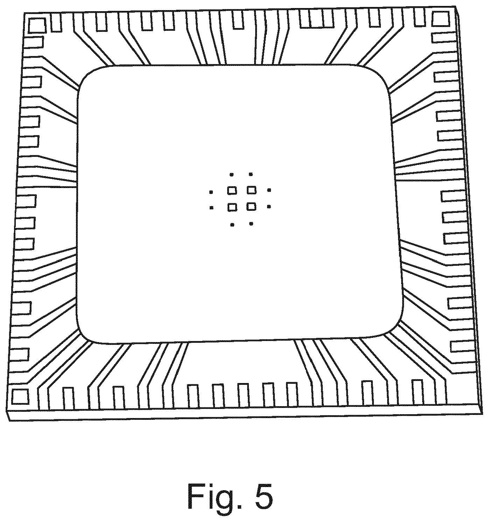

Fig. 5 stellt ein Beispiel eines praktisch nutzbaren Substrats mit geöffneten Elektroden und Lagen elektrisch leitfähigen Materials in der Passivierung gemäß dem Ausführungsbeispiel dar. Die elektrisch isolierende Seitenwandpassivierung erlaubt insbesondere vorteilhaft die Herstellung (flexibler) Implantate mit Lagen oder Flächen elektrisch leitfähigen Materials, beispielsweise Metallflächen, in der Passivierung wie in Fig. 5 gezeigt.

Erkennbar in Fig. 5 sind mehrere Öffnungen 15 unterschiedlicher Größe, angeordnet in Kreuzform mit innenliegend größeren Öffnungen 15 und außenliegend diesen gegenüber kleineren Öffnungen 15. Die geöffneten Elektroden 60 in der Mitte des Substrates sind elektrisch von den Flächen elektrisch leitfähigen Materials isoliert und dadurch einzeln nutzbar.

Wie vorstehend beschrieben wurde, wird bei einem Verfahren zur Isolation einer Passivierung entlang des Umfangs zumindest einer in vorbestimmter Weise geschaffenen Öffnung in der Passivierung eine isolierende Wandung angeordnet. Ein resultierendes Passivierungssystem weist eine elektrisch und/oder fluiddicht und/oder gasdicht isolierende Wandung entlang des Umfangs zumindest der in vorbestimmter Weise geschaffenen Öffnung in der Passivierung auf. Die isolierende Wandung einer vorbestimmten Dicke entlang eines Umfangs einer in vorbestimmter Weise erzeugten Öffnung in einer Passivierung eines medizinischen Implantats leistet vorteilhaft eine Isolierung der Passivierung oder Komponenten derselben gegen aus der Öffnung auf die Passivierung oder die Komponenten derselben einwirkende Elektrizität und/oder Flüssigkeit.

Durch das Beschichten der Seitenwände von (Elektroden-)Öffnungen mit einem elektrisch isolierenden Layer ergeben sich vielfältige Anwendungsbeispiele. Beispielsweise kann die eigentliche Passivierungsschicht ebenfalls elektrisch genutzt werden, da diese isoliert von einer künstlich geschaffenen (Elektroden-)Öffnung arbeitet, gleichzeitig aber auch gegenüber äußeren Einflüssen geschützt ist, die aufgrund dieser Öffnungen einwirken können.

Es versteht sich, dass die Erfindung nicht auf eine konkrete isolierende Schicht in einer Elektrodenöffnung einer Verkapselungs- und/oder Passivierungsstruktur an einem Implantat beschränkt ist, sondern dass verschiedenartige Konfigurationen, äquivalente Ausführungsformen und Modifikationen denkbar sind.

Es versteht sich darüber hinaus, dass den beschriebenen Ausführungsbeispielen und nicht maßstabsgetreuen Zeichnungen lediglich beispielhafter Charakter zukommt und sich insoweit für den Fachmann Modifikationen ohne Weiteres ergeben können, ohne dass dadurch der beschreibungsgemäße Rahmen und der durch die beigefügten Ansprüche definierte Schutzumfang verlassen werden. Ebenso unterliegen äußere Formen, Dimensionen und dergleichen keinen besonderen Beschränkungen, solange durch sie die erfindungsgemäße Wirkung und Funktionalität bereitgestellt und erzielt wird.

## Patentansprüche

1. Verfahren zur Isolation eines mehrschichtigen Passivierungssystems für ein medizinisches Implantat, das Passivierungssystem aufweisend eine Passivierung (10) mit zumindest einer elektrisch aktiven Zwischenlage (20, 40); zumindest eine in vorbestimmter Weise geschaffene rundförmige, rechteckförmige, elliptische oder konusförmige Öffnung (15) in der Passivierung (10);
das Verfahren aufweisend
Anordnen einer isolierenden Wandung (30) entlang des Umfangs der Öffnung (15), wobei die isolierende Wandung (30) die gesamte Seitenwandungsfläche der Öffnung (15) in der Passivierung (10) bedeckend aufbeschichtet wird, und wobei die isolierende Wandung (30) die zumindest eine elektrisch aktive Zwischenlage (20, 40) von zumindest einer Elektrode (60) eines flexiblen oder starren biostabilen, biokompatiblen und elektrisch isolierend verkapselten medizinischen Implantats als eine Bodenfläche der Öffnung (15) elektrisch und/oder fluiddicht und/oder gasdicht isoliert.

2. Verfahren nach Anspruch 1, bei dem die isolierende Wandung (30) elektrisch isolierend und/oder fluiddicht und/oder gasdicht isolierend erzeugt wird.

3. Verfahren nach Anspruch 1 oder 2, bei dem sich die isolierende Wandung (30) von einem unteren Ende der Passivierung (10) durch die Passivierung (10) hindurch zu einem oberen Ende der Passivierung (10) hin erstreckt.

4. Verfahren nach einem der vorangehenden Ansprüche, bei dem die isolierende Wandung (30) mit einer vorbestimmten Dicke erzeugt und dazu konfiguriert wird, in der Öffnung (15) einen Öffnungskanal eines vorbestimmten Durchmessers in der Passivierung (10) für eine Fluidzuleitung zu einer Elektrode (60) und/oder für eine Aufnahme einer Durchführung, einer Steckverbindung und/oder einer Headervorrichtung (50) zu erhalten und gleichzeitig elektrisch aktive Komponenten in der Passivierung (10) gegenüber dem Öffnungskanal elektrisch isoliert zu halten und/oder gegen ein Eindringen des Fluids in die Passivierung (10) zu schützen.

5. Mehrschichtiges Passivierungssystem für ein medizinisches Implantat aufweisend
eine Passivierung (10) mit zumindest einer elektrisch aktiven Zwischenlage (20, 40); zumindest eine in vorbestimmter Weise geschaffene rundförmige, rechteckförmige, elliptische oder konusförmige Öffnung (15) in der Passivierung (10);
elektrisch isolierend und/oder fluiddicht und/oder gasdicht isolierend erzeugte isolierende Wandung (30) entlang des Umfangs der Öffnung (15), wobei die isolierende Wandung (30) die gesamte Wandungsfläche der Öffnung (15) in der Passivierung (10) bedeckend angeordnet ist, und wobei die isolierende Wandung (30) dazu angeordnet und ausgeführt ist, die zumindest eine elektrisch aktive Zwischenlage (20, 40) von zumindest einer Elektrode (60) eines flexiblen oder
starren biostabilen, biokompatiblen und elektrisch isolierend verkapselten medizinischen Implantats als eine Bodenfläche der Öffnung (15) elektrisch und/oder fluiddicht und/oder gasdicht zu isolieren.

6. Passivierungssystem nach Anspruch 5, bei der sich die isolierende Wandung (30) von einem unteren Ende der Passivierung (10) durch die Passivierung (10) hindurch zu einem oberen Ende der Passivierung (10) hin erstreckt.

7. Passivierungssystem nach Anspruch 5 oder 6, bei der die isolierende Wandung (30) mit einer vorbestimmten Dicke erzeugt und dazu konfiguriert ist, in der Öffnung (15) einen Öffnungskanal eines vorbestimmten Durchmessers in der Passivierung (10) für eine Fluidzuleitung zu der Elektrode (60) und/oder eine Aufnahme einer Durchführung, einer Steckverbindung und/oder einer Headervorrichtung (50) zu erhalten und gleichzeitig elektrisch aktive Komponenten in der Passivierung (10) gegenüber dem Öffnungskanal elektrisch isoliert zu halten und/oder gegen ein Eindringen des Fluids in die Passivierung (10) zu schützen.

## Claims

1. A method for isolating a multi-layer passivation system for a medical implant, wherein the passivation system comprises
a passivation (10) with at least one electrically active intermediate layer (20, 40);
at least one round-shaped, rectangular-shaped, elliptic or cone-shaped opening (15) created in a predetermined way in the passivation (10),
the method comprising
arranging an isolating wall (30) along the circumference of the opening (15), wherein the isolating wall (30) is coated covering the entire side wall face of the opening (15) in the passivation (10), and wherein the isolating wall (30) isolates in an electrical and/or fluid-tight and/or gas-tight manner the at least one electrically active intermediate layer (20, 40) of at least one electrode (60) of a flexible or rigid medical implant which is bio-stable, bio-compatible and encapsulated in an electrically isolating manner as a bottom face of the opening (15).

2. The method according to claim 1, wherein the isolating wall (30) is produced to be electrically isolating and/or fluid-tightly and/or gas-tightly isolating.

3. The method according to claim 1 or 2, wherein the isolating wall (30) extends from a lower end of the passivation (10) through the passivation (10) to an upper end of the passivation (10).

4. The method according to one of the preceding claims, wherein the isolating wall (30) is produced with a predetermined thickness and is configured to obtain, in the opening (15), an opening channel of a predetermined diameter in the passivation (10) for a fluid supply to an electrode (60) and/or for an accommodation of a lead-through, a plug connection and/or a header device (50), and to simultaneously keep electrically active components in the passivation (10) electrically isolated with respect to the opening channel and/or protect electrically active components in the passivation (10) from penetration of the fluid in the passivation (10).

5. A multi-layer passivation system for a medical implant, comprising a passivation (10) with at least one electrically active intermediate layer (20, 40);
at least one round-shaped, rectangular-shaped, elliptic or cone-shaped opening (15) created in a predetermined way in the passivation (10),
an isolating wall (30) produced to be electrically isolating and/or fluid-tightly and/or gas-tightly isolating along the circumference of the opening (15), wherein the isolating wall (30) is arranged covering the entire wall face of the opening (15) in the passivation (10), and wherein the isolating wall (30) is arranged and configured to isolate in an electrical and/or fluid-tight and/or gas-tight manner the at least one electrically active intermediate layer (20, 40) of at least one electrode (60) of a flexible or rigid medical implant which is bio-stable, bio-compatible and encapsulated in an electrically isolating manner as a bottom face of the opening (15).

6. The passivation system according to claim 5, wherein the isolating wall (30) extends from a lower end of the passivation (10) through the passivation (10) to an upper end of the passivation (10).

7. The passivation system according to of claim 5 or 6, wherein the isolating wall (30) is produced with a predetermined thickness and is configured to obtain, in the opening (15), an opening channel of a predetermined diameter in the passivation (10) for a fluid supply to the electrode (60) and/or an accommodation of a lead-through, a plug connection and/or a header device (50), and to simultaneously keep electrically active components in the passivation (10) electrically isolated with respect to the opening channel and/or protect electrically active components in the passivation (10) from penetration of the fluid in the passivation (10).

## Revendications

1. Procédé d'isolation d'un système de passivation multicouche destiné à un implant médical, le système de passivation présentant
une passivation (10) dotée d'au moins une couche intermédiaire électriquement active (20, 40) ;
au moins une ouverture ronde, rectangulaire, elliptique ou conique créée de manière prédéterminée (15) dans la passivation (10) ;
le procédé présentant
l'agencement d'une paroi isolante (30) le long du périmètre de l'ouverture (15), dans lequel la paroi isolante (30) est revêtue pour recouvrir l'intégralité de la surface de paroi latérale de l'ouverture (15) dans la passivation (10), et dans lequel la paroi isolante (30) isole électriquement et/ou de manière étanche aux fluides et/ou de manière étanche aux gaz l'au moins une couche intermédiaire électriquement active (20, 40) de l'au moins une électrode (60) d'un implant médical souple ou rigide, biostable, biocompatible et encapsulé de manière électriquement isolante comme une surface inférieure de l'ouverture (15).

2. Procédé selon la revendication 1, pour lequel la paroi isolante (30) est produite de manière électriquement isolante et/ou de manière étanche aux fluides et/ou de manière étanche aux gaz.

3. Procédé selon la revendication 1 ou 2, pour lequel la paroi isolante (30) s'étend d'une extrémité inférieure de la passivation (10) à travers la passivation (10) à une extrémité supérieure de la passivation (10).

4. Procédé selon l'une quelconque des revendications précédentes, pour lequel la paroi isolante (30) est produite avec une épaisseur prédéterminée et configurée pour maintenir dans l'ouverture (15) un canal d'ouverture d'un diamètre prédéterminé dans la passivation (10) pour une conduite de fluide vers une électrode (60) et/ou pour une réception d'une réalisation, d'un raccordement et/ou d'un dispositif d'en-tête (50) et simultanément pour maintenir électriquement isolés des composants électriquement actifs dans la passivation (10) par rapport au canal d'ouverture et/ou pour protéger contre la pénétration du fluide dans la passivation (10).

5. Système de passivation multicouche destiné à un implant médical présentant
une passivation (10) dotée d'au moins une couche intermédiaire électriquement active (20, 40) ;
au moins une ouverture ronde, rectangulaire, elliptique ou conique créée de manière prédéterminée (15) dans la passivation (10) ;
une paroi isolante (30) produite de manière isolante, de manière électriquement isolante et/ou de manière étanche aux fluides et/ou de manière étanche aux gaz le long du périmètre de l'ouverture (15),
dans lequel la paroi isolante (30) est disposée de manière à recouvrir l'intégralité de la surface de paroi de l'ouverture (15) dans la passivation (10), et dans lequel
la paroi isolante (30) est disposée et conçue pour isoler électriquement et/ou de manière étanche aux fluides et/ou de manière étanche aux gaz l'au moins une couche intermédiaire électriquement active (20, 40) de l'au moins une électrode (60) d'un implant médical souple ou rigide, biostable, biocompatible et encapsulé de manière électriquement isolante comme une surface inférieure de l'ouverture (15).

6. Système de passivation selon la revendication 5, pour lequel la paroi isolante (30) s'étend d'une extrémité inférieure de la passivation (10) à travers la passivation (10) à une extrémité supérieure de la passivation (10).

7. Procédé selon la revendication 5 ou 6, pour lequel la paroi isolante (30) est produite avec une épaisseur prédéterminée et configurée pour maintenir dans l'ouverture (15) un canal d'ouverture d'un diamètre prédéterminé dans la passivation (10) pour une conduite de fluide vers une électrode (60) et/ou pour une réception d'une réalisation, d'un raccordement et/ou d'un dispositif d'en-tête (50) et simultanément pour conserver électriquement isolés des composants électriquement actifs dans la passivation (10) par rapport au canal d'ouverture et/ou pour protéger contre une pénétration du fluide dans la passivation (10).
